# EUROPÄISCHE PATENTSCHRIFT

(11) **EP 0 388 750 B1**
(45) Veröffentlichungstag und Bekanntmachung des Hinweises auf die Patenterteilung: **26.01.1994**
(21) Anmeldenummer: 90104645.8
(22) Anmeldetag: 12.03.1990
(51) Int. Cl.: C07D 409/04, A61K 31/50, C07D 333/24

(54) **Thienylpiperazine, Verfahren zu ihrer Herstellung, ihre Verwendung sowie diese enthaltende Arzneimittel und deren Herstellung**
Thienyl piperazines, process for their preparation, their use, pharmaceutical agents containing them and their production
Thiényl-pipérazines, leur procédé de préparation, leur application ainsi que les médicaments les contenant et leur production

(30) Priorität: 22.03.1989 DE 3909379
(43) Veröffentlichungstag der Anmeldung: 26.09.1990
(73) Patentinhaber: CASSELLA Aktiengesellschaft, D-60386 Frankfurt (DE)
(72) Erfinder: Schönafinger, Karl, Dr., D-8755 Alzenau (DE); Beyerle, Rudi, Dr., D-6000 Frankfurt am Main 60 (DE); Schindler, Ursula, Dr., D-6238 Hofheim/Ts. (DE); Jablonka, Bernd, Dr., D-6232 Bad Soden (DE); Troke, Jeffery, Dr., Newport Pagnell, Buckinghamshire (GB)
(74) Vertreter: Muley, Ralf, Dr.

(56) Entgegenhaltungen:
- EP-A- 0 072 932
- EP-A- 0 181 152
- EP-A- 0 342 536
- DE-A- 3 329 028

## Beschreibung

Die vorliegende Erfindung betrifft Thienylpiperazine der allgemeinen Formel I 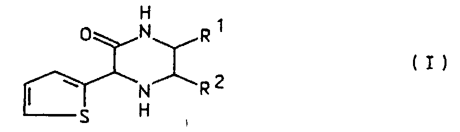
worin R¹ Wasserstoff oder doppelt gebundener Sauerstoff und R² Wasserstoff oder doppelt gebundener Sauerstoff bedeuten, wobei R¹ und R² nicht gleichzeitig die gleiche Bedeutung besitzen, sowie ihre pharmazeutisch verträglichen Salze. Somit umfaßt die vorliegende Erfindung 3-(Thien-2-yl)-piperazin-2,5-dion der Formel Ia 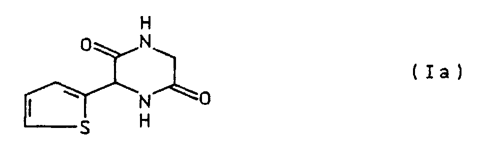
und 3-(Thien-2-yl)-piperazin-2,6-dion der Formel Ib 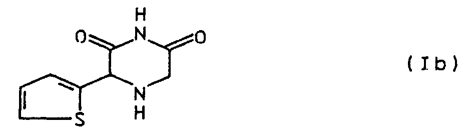
sowie pharmazeutisch verträgliche Salze davon. Pharmazeutisch verträgliche Salze sind bevorzugt Säureadditionssalze, besonders bevorzugt die Hydrochloride. Die Verbindungen der allgemeinen Formel I können hergestellt werden durch Cyclisierung der Verbindung der Formel II 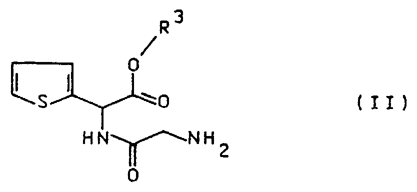
worin R³ (C₁-C₄)-Alkyl bedeutet, beziehungsweise durch Cyclisierung der Verbindung der Formel III 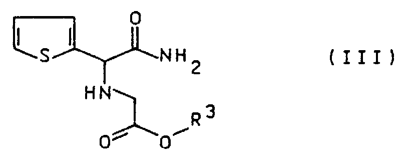
worin R³ wie oben definiert ist, und gegebenenfalls anschließende Überführung in ein pharmazeutisch verträgliches Salz.

Die Cyclisierungen der Verbindungen der Formeln II und III erfolgen bevorzugt in bekannter Weise durch Erwärmen in einem geeigneten Lösungsmittel oder durch die katalytische Wirkung von Säuren oder Basen.

Geeignete Lösungsmittel sind insbesondere Alkohole, wie zum Beispiel Methanol, Ethanol, Isopropanol, Butanol, Glykol, Ether, wie zum Beispiel Dimethoxyethan oder Diethoxyethan sowie Toluol und Xylol.

Bevorzugt wird auf 40°C bis zum Siedepunkt des Lösungsmittels erwärmt.

Zur Cyclisierung geeignete Säuren oder Basen sind insbesondere Chlorwasserstoff, Trifluoressigsäure, Toluolsulfonsäure, Natriumacetat, Natriumcarbonat, Natriumhydroxid, Natriumhydrid, Natriumamid.

Ausgangspunkte für die Herstellung der Verbindungen der Formel II sind die Thienylglycinester der allgemeinen Formel IV 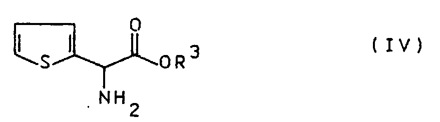
worin R³ wie oben definiert ist. Diese werden durch Umsetzung mit dem gemischten Anhydrid der Formel V 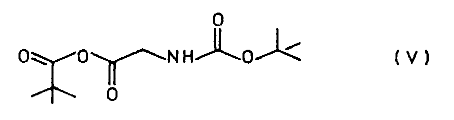
und anschließender Hydrolyse in an sich bekannter Weise in die Verbindungen der Formel II überführt.

Die Alkylierung mit dem gemischten Anhydrid wird bevorzugt in inerten Lösungsmitteln, besonders bevorzugt in DMF oder Ether, ausgeführt. Die Reaktionstemperaturen liegen bevorzugt bei -20 bis +60°C, besonders bevorzugt bei -20°C bis Raumtemperatur. Die Hydrolyse geschieht bevorzugt sauer, besonders bevorzugt mit Chlorwasserstoff oder Trifluoressigsäure. Die Reaktionstemperaturen liegen hier bevorzugt bei 0 bis 50°C, besonders bevorzugt bei Raumtemperatur.

In einer bevorzugten Ausführungsform wird das Reaktionsprodukt der Umsetzung zwischen der Verbindung der allgemeinen Formel IV und dem gemischten Anhydrid der Formel V direkt, das heißt ohne Isolierung der Verbindung der allgemeinen Formel II, in die erfindungsgemäße Verbindung der Formel Ia überführt.

Ausgangsprodukt für die Herstellung der Verbindungen der Formel II ist das Thienylglycinamid der Formel VI 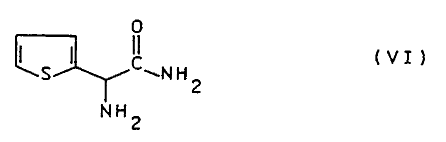
das durch Umsetzung mit Halogenessigsäureestern der allgemeinen Formel VII 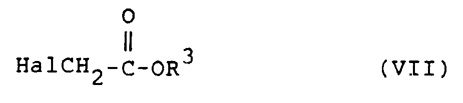
worin Hal Halogen, bevorzugt Brom, bedeutet und R³ wie oben definiert ist, in an sich bekannter Weise direkt zu den gewünschten Produkten führt. Bei Reaktionstemperaturen von vorzugsweise 0 - 100°C werden dabei bevorzugt inerte Lösungsmittel, besonders bevorzugt Ether, DMF oder DME verwendet.

Die Verbindungen der Formeln IV und VI sind beispielsweise aus der käuflichen 2-Thienylglyoxylsäure durch Veresterung bzw. Amidierung bzw. aus deren Ester, wie beispielsweise aus 2-Thienylglyoxylsäureethylester, durch Umesterung bzw. Ammonolyse mit Ammoniak, anschließende Umsetzung mit Hydroxylamin zum entsprechenden Oxim und Reduktion des Oxims zugänglich. Alternativ dazu können die Verbindungen der Formeln IV und VI auch durch Veresterung bzw. Amidierung von käuflichem 2-Thienylglycin hergestellt werden.

Veresterungen, Umesterungen, Amidierungen, Ammonolyse, Oximbildung sowie Reduktion sind Reaktionstypen, die dem Fachmann an sich bekannt und in allen gängigen Lehrbüchern der Organischen Chemie, beispielsweise in Houben-Weyl, "Methoden der Organischen Chemie", beschrieben sind.

Die Verbindung der Formel V ist bekannt und wird vorteilhafterweise in situ aus N-tert.Butoxycarbonylglycin und Trimethylessigsäurechlorid hergestellt.

Sofern die erfindungsgemäßen Verbindungen der allgemeinen Formel I basische Reste enthalten, bilden sie mit anorganischen oder organischen Säuren Säureadditionssalze. Zur Bildung derartiger Säureadditionssalze sind anorganische und organische Säuren geeignet. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungsmittel oder Verdünnungsmittel, hergestellt. Bei der Synthese der Verbindungen der allgemeinen Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Sofern die erfindungsgemäßen Verbindungen der allgemeinen Formel I, saure Gruppen aufweisen, können diese auch in Salzform vorliegen. Bevorzugt sind die Natrium-, Kalium-und Ammoniumsalze, die durch Umsetzung der sauren Form mit entsprechenden Basen erhalten werden können.

Die erfindungsgemäßen Verbindungen der allgemeinen Formel I und ihre pharmazeutisch verträglichen Salze besitzen wertvolle pharmakologische Eigenschaften. Sie sind zentralwirksam, beispielsweise zeigen sie encephalotrope und nootrope Wirkungen und dienen zur Behandlung von Krankheiten der Gehirnfunktionen wie cerebraler Insuffizienz, cerebraler Alterungsvorgänge, verminderter Gedächtnisleistung wie sie auch bei der Alzheimer Krankheit oder der Multi-Infarkt Demenz oder bei verminderter Lernleistung auftreten. Sie sind den bisher bekannten Verbindungen gleicher Wirkungsrichtung überraschenderweise erheblich überlegen. Sie zeigen eine ausgezeichnete Wirksamkeit in verschiedenenartigen Tests, wie z.B. bei der Verlängerung der Überlebenszeit unter Natriumnitrit-Hypoxie nach Gibsen und Bless (J. Neurochemistry 27, (1976)), bei der Verbesserung der stickstoffinduzierten Hypoxietoleranz, wobei Versuchstiere nach Prämedikation mit den untersuchten Präparaten mit reinem Stickstoff beatmet werden und die Verlängerung des Zeitraumes zwischen Einsatz der Beatmung und elektrischer Neutralität des Elektroencephalogramms sowie die Letalität gemessen werden.

Auch in Tests, die direkt auf die Erfassung der Lern- und Gedächtnisleistung abzielen, wie z.B. den bekannten "avoidance"-Tests, sind die erfindungsgemäßen Produkte sehr gut wirksam.

Die Prüfung in den genannten und einer Reihe weiterer Tests ergibt, daß die erfindungsgemäßen Verbindungen in niedrigen Dosen bei geringer Toxizität überraschenderweise ein besonders günstiges, bei bekannten Präparaten in dieser Form nicht vorliegendes Wirkprofil aufweisen. Dies soll anhand folgender Tabellen präzisiert werden:

### 1) Scopolamin-induzierte Amnesie bei der Maus

Die Injektion einer Scopolamin Dosis von 3 mg/kg i.p. führt zu einer Amnesie in einem Passive-avoidance Lernversuch. Die perorale Vorbehandlung mit den Verbindungen der allgemeinen Formel I kann, wie die folgende Tabelle zeigt, die experimentell induzierte Amnesie signifikant antagonisieren. Die bekannte Substanz Piracetam ist als Vergleich dazu nur marginal wirksam.

| Verbindung der Formel | Dosis mg/kg po | Scopolamin-Amnesie % Umkehr der Amnesie |
|---|---|---|
| Ia | 30 | 53 |
| Ib | 30 | 59 |
| Piracetam (Vergleich) | 30 | 18 |

### 2) Ischämie-induzierte Amnesie beim mongolischen Gerbil

Eine kurzdauernde bilaterale cerebrale Ischämie (3-5 min) führt beim Gerbil zu einer anhaltenden Lernstörung des Passive-avoidance Lernens. Werden die Tiere sofort nach dem Ende der Ischämie intraperitoneal mit den Verbindungen der allgemeinen Formel I behandelt, kann die Lernstörung antagonisiert werden. In der nachfolgenden Tabelle sind diejenigen Dosierungen angegeben, die eine halbmaximale (ca. 50%ige) Antagonisierung der Lernstörung hervorrufen. Die erfindungsgemäßen Verbindungen zeigen eine ausgeprägte Überlegenheit über Verbindungen des Standes der Technik.

| Verbindung der Formel | Dosis mg/kg ip | Ischämie-induzierte Lernstörung % Umkehr der Lernst. |
|---|---|---|
| Ia | 1,00 | 46 |
| Ib | 3,00 | 46 |
| Aniracetam (Vergleich) | 30,00 | 45 |
| Oxiracetam (Vergleich) | 100,00 | 46 |

Die Verbindungen der allgemeinen Formel I und ihre physiologisch verträglichen Salze stellen somit eine Bereicherung der Pharmazie dar.

Die erfindungsgemäßen Verbindungen der allgemeinen Formel I und ihre pharmazeutisch verträglichen Salze können daher am Menschen als Heilmittel z.B. bei der Bekämpfung bzw. Vorbeugung von Erkrankungen, die durch eine Einschränkung der Gehirnfunktion bedingt sind und bei der Behandlung und Vorbeugung von cerebralen Alterungsvorgängen Anwendung finden.

Die Verbindungen der allgemeinen Formel I und ihre pharmazeutisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glycerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Isosorbidmononitrat, Glycerolnitrat, Molsidomin und Verapamil, ß-Blocker, wie Propranolol, Oxprenolol, Atenolol, Metoprolol und Penbutolol. Darüberhinaus lassen sich die Verbindungen mit anderen nootrop wirksamen Substanzen, wie z.B. Piracetam, oder ZNS-aktiven Substanzen, wie Pirlindol, Sulpirid etc. kombinieren.

Die Dosierung kann innerhalb weiter Grenzen variieren und ist in jedem einzelnen Fall den individuellen Gegebenenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,1 bis 50 mg, vorzugsweise 0,5 bis 10 mg Wirkstoff der allgemeinen Formel I oder eines pharmakologisch annehmbaren Salzes davon pro Dosis.

Die folgenden Beispiele 1 und 2 betreffen die Herstellung von Verbindungen der allgemeinen Formel I, die Beispiele A bis H betreffen die Herstellung von Zubereitungen der Verbindungen der allgemeinen Formel I.

### Beispiel 1

### 3-(Thien-2-yl)-piperazin-2,5-dion

### a) Thien-2-yl-aminoessigsäuremethylester HCl

In die Lösung von 6,8 g Thien-2-yl-aminoessigsäure in 70 ml Methanol wird HCl-Gas eingeleitet und die Mischung 6 h auf 50°C erwärmt. Das überschüssige Methanol und die Salzsäure werden im Vakuum abgezogen und der feste Rückstand mit Methanol gewaschen und im Vakuum getrocknet.
Ausbeute: 5,9 g FP.: 179°C

### b) Thien-2-yl-N-tert.butoxycarbonylaminoacetyl-aminoessigsäuremethylester

N-tert.Butoxycarbonyl-glycin (4,3 g) und 2,5 g Triethylamin werden in 240 ml Tetrahydrofuran gelöst und auf -20°C abgekühlt. Dann werden 2,9 g Trimethylessigsäurechlorid zugetropft und 30 min bei -20°C nachgerührt. Zu dieser Mischung wird die Lösung von 5,6 g der Verbindung aus Beispiel 1a) und 2,5 g Triethylamin in 20 ml Dimethylformamid getropft. Das Kältebad wird entfernt, und die Mischung wird 2 h bei Raumtemperatur gerührt. Der Niederschlag wird abgesaugt, das Filtrat eingeengt. Der ölige Rückstand wird in Essigester gelöst, zweimal mit je 100 ml Wasser, einmal mit 100 ml 5%iger Natriumbicarbonatlösung und einmal mit 100 ml 1 N Zitronensäure gewaschen, über Magnesiumsulfat getrocknet. Nach dem Entfernen des Lösungsmittels verbleibt ein öliges Produkt.
Ausbeute: 6,4 g Öl

### c) 3-(Thien-2-yl)-piperazin-2,5-dion

Die Mischung von 6,4 g Thien-2-yl-N-(tert.butoxycarbonylamino-acetyl)-amino-essigsäuremethylester und 200 ml Ameisensäure wird 15 h bei Raumtemperatur gerührt. Die flüchtigen Anteile werden im Vakuum abgezogen, und der Rückstand wird in einer Mischung aus 80 ml Toluol und 300 ml Butanol 3 h am Rückfluß zum Sieden erhitzt. Dann werden die Lösungsmittel am Rotationsverdampfer entfernt und der Rückstand aus Methanol umkristallisiert.
Ausbeute : 1,5 g Fp: 220 bis 222°C

### Beispiel 2

### 3-(Thien-2-yl)-piperazin-2,6-dion

### a) Thien-2-yl-glyoxyl-säure-amid

In die Lösung von 73,6 g Thien-2-yl-glyoxylsäureethylester werden unter Kühlung 13,6 g Ammoniak eingeleitet und die erhaltene Mischung 2 h bei Raumtemperatur gerührt Das Lösungsmittel wird im Vakuum abgezogen und der Rückstand aus Wasser:Ethanol (80:20) umkristallisiert.
Ausbeute: 40,3 g Fp.: 86 - 88°C

### b) Thien-2-yl-hydroximino-essigsäure-amid

Die Mischung aus 15,5 g Thien-2-yl-glyoxylsäureamid, 75 ml Wasser, 75 ml Ethanol, 10,4 g Hydroxylaminhydrochlorid und 24,6 g Natriumacetat wird 5 h bei 40°C gerührt, die flüchtigen Anteile werden im Vakuum abgezogen und der Rückstand in Wasser aufgenommen. Das Produkt wird mit Essigester extrahiert, die organische Phase getrocknet und eingeengt. Der Rückstand wird aus wenig Essigester umkristallisiert.
Ausbeute: 5,2 g Fp.: 146 - 149°C

### c) Thien-2-yl-aminoessigsäureamid

Die Mischung aus 5,1 g Thien-2-yl-hydroximinoessigsäureamid und 180 ml Methanol wird in Gegenwart von Raney-Nickel bei 100°C/50 bar hydriert. Nach Beendigung der Wasserstoffaufnahme wird vom Ramy Nickel abgesaugt, das Filtrat eingeengt und der Rückstand aus Diethylether umkristallisiert.
Ausbeute: 2,6 g Fp.: 82 - 84°C

### d) Thien-2-yl-N-methoxycarbonylmethyl-aminoessigsäureamid

Die Lösung von 9,4 g Thien-2-yl-aminoessigsäureamid (Beispiel 2c), 10,1 g Bromessigsäuremethylester und 6,7 g Triethylamin in 100 ml Dimethoxyethan wird 16 h zum Sieden erhitzt. Der nach dem Einengen verbleibende Rückstand wird in Wasser/Essigester verteilt. Die Essigesterphase wird getrocknet, mit Aktivkohle aufgekocht, filtriert und eingeengt. Der Rückstand wird aus Essigester umkristallisiert.
Ausbeute: 2,7 g

### e) 3-(Thien-2-yl)-piperazin-2,6-dion

Zur Lösung von 4,6 g Thien-2-yl-N-methoxycarbonylmethylamino-essigsäureamid in 50 ml wasserfreiem Xylol wird Natriumhydrid (1,6 g; 50%ig in Weißöl) portionsweise zugefügt und das Gemisch 15 h zum Sieden erhitzt. Nach Zugabe von 1,8 g Eisessig wird heiß über Celite abgesaugt, das Filtrat im Eisbad abgekühlt und der Feststoff abgesaugt, mit Ether gewaschen und getrocknet.
Ausbeute: 1,0 g Fp: 128 bis 129°C

### Beispiel A

Emulsionen mit 3 mg Wirkstoff per 5ml können nach folgender Rezeptur hergestellt werden:

| | |
|---|---|
| Wirkstoff | 0,06g |
| Neutralöl | q.s. |
| Natriumcarboxymethylzellulose | 0,6g |
| Polyoxyethylenstearat | q.s. |
| Reinglyzerin | 0,2 bis 2g |
| Aromastoffe | q.s. |
| Wasser (entmineralisiert oder destilliert) | ad 100 ml |

### Beispiel B

Tabletten können nach folgender Formulierung hergestellt werden:

| | |
|---|---|
| Wirkstoff | 2mg |
| Lactose | 60 mg |
| Maisstärke | 30 mg |
| lösliche Stärke | 4mg |
| Magnesiumstearat | 4mg |
| | 1̅0̅0̅m̅g̅ |

### Beispiel C

Für die Herstellung von Weichgelatinekapseln mit 5mg Wirkstoff pro Kapsel eignet sich die folgende Zusammensetzung:

| | |
|---|---|
| Wirkstoff | 5mg |
| Mischung von Triglyzeriden aus Kokosöl | 150mg |
| Kapselinhalt | 1̅5̅5̅m̅g̅ |

### Beispiel D

Für die Herstellung von Dragees eignet sich folgende Formulierung

| | |
|---|---|
| Wirkstoff | 3mg |
| Maisstärke | 100mg |
| Lactose | 55mg |
| sec Calciumphosphat | 30mg |
| lösliche Stärke | 3mg |
| Magnesiumstearat | 5mg |
| kolloidale Kieselsäure | 4mg |
| | 2̅0̅0̅m̅g̅ |

### Beispiel E

Dragees, enthaltend einen erfindungsgemäßen Wirkstoff und einen anderen therapeutisch wirksamen Stoff:

| | |
|---|---|
| Wirkstoff | 6mg |
| Propranolol | 40mg |
| Milchzucker | 90mg |
| Maisstärke | 90mg |
| sec Calciumphosphat | 34mg |
| lösliche Stärke | 3mg |
| Magnesiumstearat | 3mg |
| kolloidale Kieselsäure | 4mg |
| | 2̅7̅0̅m̅g̅ |

### Beispiel F

Dragees, enthaltend einen erfindungsgemäßen Wirkstoff und einen anderen therapeutisch wirksamen Stoff:

| | |
|---|---|
| Wirkstoff | 5mg |
| Pirlindol | 5mg |
| Milchzucker | 60mg |
| Maisstärke | 90mg |
| sec Calciumphosphat | 30mg |
| lösliche Stärke | 3mg |
| Magnesiumstearat | 3mg |
| kolloidale Kieselsäure | 4mg |
| | 2̅0̅0̅m̅g̅ |

### Beispiel G

Kapseln, enthaltend einen erfindungsgemäßen Wirkstoff und einen anderen therapeutisch wirksamen Stoff:

| | |
|---|---|
| Wirkstoff | 5mg |
| Nicergolin | 5mg |
| Maisstärke | 185mg |
| | 1̅9̅5̅m̅g̅ |

### Beispiel H

Injektionslösungen mit 1mg Wirkstoff pro ml können nach folgender Rezeptur hergestellt werden:

| | |
|---|---|
| Wirkstoff | 1,0mg |
| Polyethylenglykol 400 | 0,3mg |
| Natriumchlorid | 2,7mg |
| Wasser zu Injektionszwecken auf | 1 ml |

## Patentansprüche (Patentansprüche für folgende(n) Vertragsstaat(en): AT, BE, CH, DE, DK, FR, GB, IT, LI, NL, SE)

1. Thienylpiperazine der allgemeinen Formel I 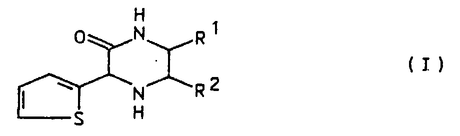 worin R¹ Wasserstoff oder doppelt gebundener Sauerstoff und R² Wasserstoff oder doppelt gebundener Sauerstoff bedeuten, wobei R¹ und R² nicht gleichzeitig die gleiche Bedeutung besitzen, sowie ihre pharmazeutisch verträglichen Salze.

2. 3-(Thien-2-yl)-piperazin-2,5-dion sowie pharmazeutisch verträgliche Salze davon.

3. 3-(Thien-2-yl)-piperazin-2,6-dion sowie pharmazeutisch verträgliche Salze davon.

4. Verfahren zur Herstellung einer Verbindung der Ansprüche 1 bis 3, dadurch gekennzeichnet, daß eine Verbindung der Formel II 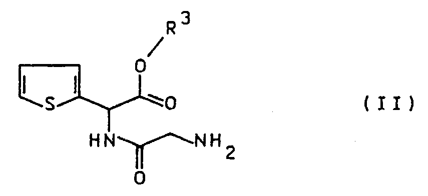 bzw. eine Verbindung der Formel III 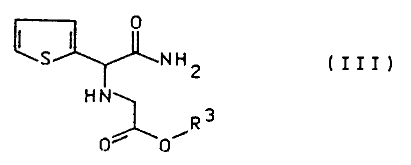 worin R³ (C₁-C₄)-Alkyl bedeutet, cyclisiert und gegebenenfalls in ein pharmazeutisch verträgliches Salz überführt wird.

5. Verwendung eines Thienylpiperazinderivats gemäß einem oder mehreren der Ansprüche 1 bis 3 zur Herstellung eines pharmazeutischen Präparates zur Bekämpfung und Vorbeugung von Erkrankungen, die durch Einschränkung der Gehirnfunktion bedingt sind, oder zur Bekämpfung von cerebralen Alterungsvorgängen.

6. Pharmazeutisches Präparat, dadurch gekennzeichnet, daß es als Wirkstoff ein oder mehrere Thienylpiperazinderivate gemäß einem oder mehreren der Ansprüche 1 bis 3 oder ein pharmazeutisch verträgliches Salz davon zusammen mit einem pharmazeutisch annehmbaren Trägerstoff und gegebenenfalls pharmazeutisch annehmbaren Zusatzstoffen und gegebenenfalls noch einen oder mehrere andere pharmakologische Wirkstoffe enthält.

7. Verfahren zur Herstellung eines pharmazeutischen Präparates gemäß Anspruch 6, dadurch gekennzeichnet, daß ein oder mehrere Thienylpiperazinderivate gemäß einem oder mehreren der Ansprüche 1 bis 3 oder ein pharmazeutisch verträgliches Salz davon durch Mischen mit geeigneten pharmazeutisch zulässigen Träger- und Zusatzstoffen und gegebenenfalls noch einer oder mehreren anderen pharmazeutisch wirksamen Substanzen in eine zur Verabreichung geeignete Form gebracht werden.

## Patentansprüche (Patentansprüche für folgende(n) Vertragsstaat(en): ES, GR)

1. Verfahren zur Herstellung von Thienylpiperazinen der allgemeinen Formel I 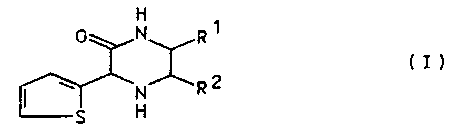 worin R¹ Wasserstoff oder doppelt gebundener Sauerstoff und R² Wasserstoff oder doppelt gebundener Sauerstoff bedeuten, wobei R¹ und R² nicht gleichzeitig die gleiche Bedeutung besitzen, sowie ihre pharmazeutisch verträglichen Salze, dadurch gekennzeichnet, daß eine Verbindung der Formel II 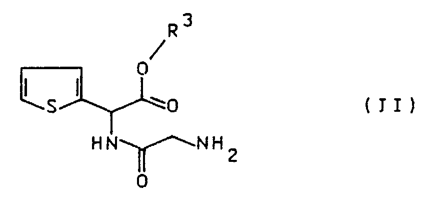 bzw. eine Verbindung der Formel III 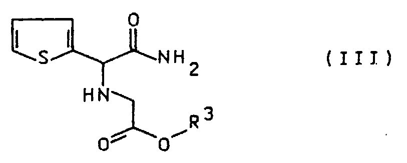 worin R³ (C₁-C₄)-Alkyl bedeutet, cyclisiert und gegebenenfalls in ein pharmazeutisch verträgliches Salz überführt wird.

2. Verfahren gemäß Anspruch 1, dadurch gekennzeichnet, daß 3-(Thien-2-yl)-piperazin-2,5-dion oder ein pharmazeutisch verträgliches Salz davon hergestellt wird.

3. Verfahren gemäß Anspruch 1, dadurch gekennzeichnet, daß 3-(Thien-2-yl)-piperazin-2,6-dion oder ein pharmazeutisch verträgliches Salz davon hergestellt wird.

4. Verwendung eines nach einem der Verfahren der Ansprüche 1 bis 3 hergestellten Thienylpiperazins der allgemeinen Formel I zur Herstellung eines pharmazeutischen Präparates zur Bekämpfung und Vorbeugung von Erkrankungen, die durch Einschränkung der Gehirnfunktion bedingt sind, oder zur Bekämpfung von cerebralen Alterungsvorgängen.

5. Verfahren zur Herstellung eines pharmazeutischen Präparates, dadurch gekennzeichnet, daß ein oder mehrere nach den Verfahren der Ansprüche 1 bis 3 hergestellten Thienylpiperazine der allgemeinen Formel I oder ein pharmazeutisch verträgliches Salz davon durch Mischen mit geeigneten pharmazeutisch zulässigen Träger- und Zusatzstoffen und gegebenenfalls noch einer oder mehreren anderen pharmazeutisch wirksamen Substanzen in eine zur Verabreichung geeignete Form gebracht werden.

## Claims (Claims for the following Contracting State(s): AT, BE, CH, DE, DK, FR, GB, IT, LI, NL, SE)

1. Thienylpiperazines of the general formula I 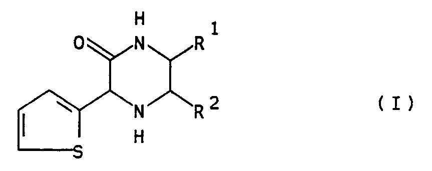 in which R¹ denotes hydrogen or double-bonded oxygen and R² denotes hydrogen or double-bonded oxygen, with R¹ and R² not both having the same meaning, as well as the pharmaceutically tolerated salts thereof.

2. 3-(2-Thienyl)piperazine-2,5-dione and pharmaceutically tolerated salts thereof.

3. 3-(2-Thienyl)piperazine-2,6-dione and pharmaceutically tolerated salts thereof.

4. Process for the preparation of a compound of Claims 1 to 3, characterized in that a compound of the formula II 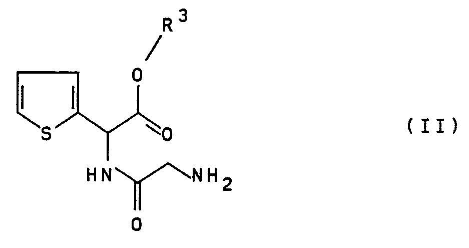 or a compound of the formula III 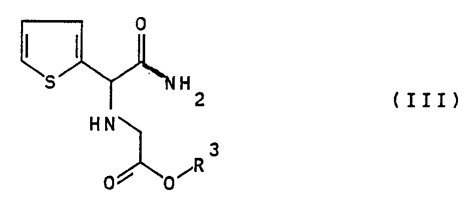 in which R³ denotes (C₁-C₄)-alkyl, is cyclized and, where appropriate, converted into a pharmaceutically tolerated salt.

5. Use of a thienylpiperazine derivative according to one or more of Claims 1 to 3 for preparing a pharmaceutical product for controlling and preventing disorders based on impairment of brain function or for controlling cerebral ageing processes.

6. Pharmaceutical product, characterized in that it contains as active compound one or more thienylpiperazine derivatives according to one or more of Claims 1 to 3 or a pharmaceutically tolerated salt thereof together with a pharmaceutically acceptable vehicle and, where appropriate, pharmaceutically acceptable additives and, where appropriate, also one or more other pharmacological active compounds.

7. Process for the preparation of a pharmaceutical product according to Claim 6, characterized in that one or more thienylpiperazine derivatives according to one or more of Claims 1 to 3 or a pharmaceutically tolerated salt thereof is converted into a form suitable for administration by mixing with suitable pharmaceutically acceptable vehicles and additives and, where appropriate, also one or more other pharmaceutically active substances.

## Claims (Claims for the following Contracting State(s): ES, GR)

1. Process for preparing thienylpiperazines of the general formula I 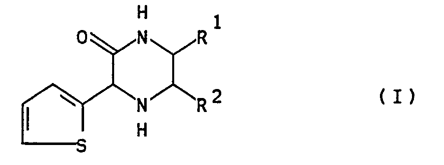 in which R¹ denotes hydrogen or double-bonded oxygen and R² denotes hydrogen or double-bonded oxygen, with R¹ and R² not both having the same meaning, as well as the pharmaceutically tolerated salts thereof, characterized in that a compound of the formula II 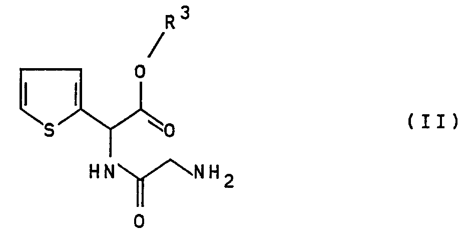 or a compound of the formula III 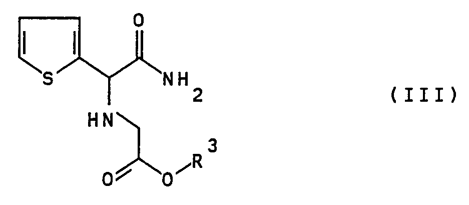 in which R³ denotes (C₁-C₄)-alkyl, is cyclized and, where appropriate, converted into a pharmaceutically tolerated salt.

2. Process according to Claim 1, characterized in that 3-(2-thienyl)piperazine-2,5-dione or a pharmaceutically tolerated salt thereof is prepared.

3. Process according to Claim 1, characterized in that 3-(2-thienyl)piperazine-2,6-dione or a pharmaceutically tolerated salt thereof is prepared.

4. Use of a thienylpiperazine of the general formula I prepared according to one of the processes of Claims 1 to 3 for preparing a pharmaceutical product for controlling and preventing disorders based on impairment of brain function or for controlling cerebral ageing processes.

5. Process for the preparation of a pharmaceutical product, characterized in that one or more thienylpiperazines of the general formula I prepared according to the processes of Claims 1 to 3 or a pharmaceutically tolerated salt thereof is converted into a form suitable for administration by mixing with suitable pharmaceutically acceptable vehicles and additives and, where appropriate, also one or more other pharmaceutically active substances.

## Revendications (Revendications pour l'(les) Etat(s) contractant(s) suivant(s): AT, BE, CH, DE, DK, FR, GB, IT, LI, NL, SE)

1. Thiénylpiperazines de formule générale (I) 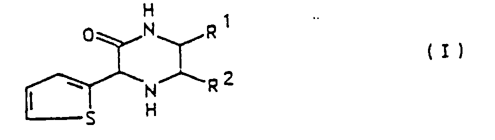 dans laquelle R¹ représente un atome d'hydrogène ou un atome d'oxygène doublement lié, et R² représente un atome d'hydrogène ou un atome d'oxygène doublement lié, les symboles R¹ et R² n'ayant pas simultanément le même sens), ainsi que leurs sels pharmaceutiquement acceptables.

2. La 3-(thién-2-yl)-pipérazine-2,5-dione et ses sels pharmaceutiquement acceptables.

3. La 3-(thién-2-yl-pipérazine-2,6-dione et ses sels pharmaceutiquement tolérables.

4. Procédé pour préparer un composé selon les revendicationS 1 à 3, caractérisé en ce qu'on cyclise un composé de formule (II) 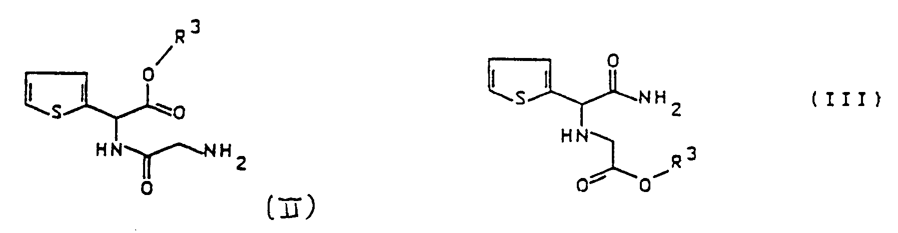 ou un composé de formule (III)
(dans lesquelles R³ représente un groupe alkyle en (C₁ à C₄) et l'on transforme éventuellement ce composé en un sel pharmaceutiquement acceptable.

5. Utilisation d'un dérivé de thiénylpipérazine selon une ou plusieurs des revendications 1 à 3 pour confectionner une préparation pharmaceutique destinée à combattre et à prévenir des maladies provoquées par une limitation du fonctionnement cérébral, ou pour combattre des processus d'altération cérébrale.

6. Préparation pharmaceutique, caractérisée en ce qu'elle contient comme substance active un ou plusieurs dérivés de thiénylpipérazine selon une ou plusieurs des revendications 1 à 3, ou un sel pharmaceutiquement admissible de ces dérivés, avec une substance de support, véhicule ou excipient pharmaceutiquement admissible et éventuellement des additifs pharmaceutiquement acceptables et éventuellement encore une ou plusieurs autre(s) substance(s) pharmacologiquement active(s)

7. Procédé pour confectionner une préparation pharmaceutique selon la revendication 6, caractérisé en ce qu'on met sous une forme convenant pour une administration un ou plusieurs dérivés de thiénylpipérazine selon une ou plusieurs des revendications 1 à 3, ou un sel pharmaceutiquement acceptable de ces dérivés, par mélangeage avec des supports, excipients ou véhicules et additifs pharmaceutiquement admissibles, et éventuellement avec encore une ou plusieurs autre(s) substance(s) pharmaceutiquement active(s).

## Revendications (Revendications pour l'(les) Etat(s) contractant(s) suivant(s): ES, GR)

1. Procédé pour produire des thiénylpipérazines de formule générale (I) 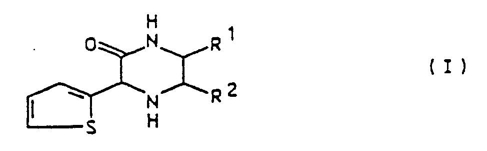 (dans laquelle R¹ représente un atome d'hydrogène ou un atome d'oxygène doublement lié, et R² représente un atome d'hydrogène ou un atome d'oxygène doublement lié, les symboles R¹ et R² n'ayant pas simultanément le même sens), ainsi que leurs sels pharmaceutiquement acceptables, procédé caractérisé en ce qu'on cyclise un composé de formule (II) 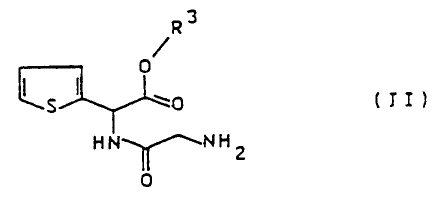 ou un composé de formule (III) 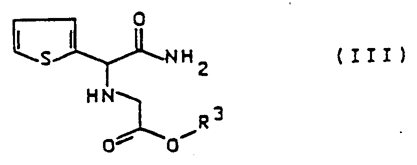 (dans laquelle R³ représente un groupe alkyle en C₁ à C₄) et éventuellement on transforme en un sel pharmaceutiquement admissible.

2. Procédé selon la revendication 1, caractérisé en ce qu'on produit la 3-(thién-2-yl)-pipérazine-2,5-dione ou un sel pharmaceutiquement admissible de cette dione.

3. Procédé selon la revendication 1, caractérisé en ce qu'on produit la 3-(thién-2-yl-pipérazine-2,6-dione ou un sel pharmaceutiquement tolérable de cette dione.

4. Utilisation d'une des thiénylpipérazines de formule générale (I), produite selon l'un des procédés des revendications 1 à 3, pour confectionner une préparation pharmaceutique destinée à combattre ou à prévenir des maladies provoquées par une limitation du fonctionnement cérébral ou pour combattre des processus d'altération cérébrale.

5. Procédé pour fabriquer une préparation pharmaceutique caractérisée en ce qu'on met sous une forme convenant bien pour une administration une ou plusieurs des thiénylpipérazines de formule générale (I) produite(s) selon les procédés des revendications 1 à 3 ou un sel pharmaceutiquement tolérable dérivant de ces thiénylpipérazines par mélangeage avec des supports, excipients ou véhicules et avec des additifs pharmaceutiquement admissibles et éventuellement avec encore une ou plusieurs autres substances pharmaceutiquement actives.
